# EUROPEAN PATENT APPLICATION

(11) **EP 4 279 589 A1**
(43) Date of publication of application: **22.11.2023**
(21) Application number: 21919019.6
(22) Date of filing: 12.11.2021
(51) Int. Cl.: C12N 11/02

(54) **MEMBRANE-SHAPED IMMOBILIZED CELL, POLYPEPTIDE, OLIGOPEPTIDE OR PROTEIN, AND PREPARATION METHOD THEREFOR**

(30) Priority: 15.01.2021 CN 202110053912
(71) Applicant: BioRight Worldwide Company Limited, Road Town, Tortola (VG)
(72) Inventor: WANG, Jun, Hong Kong Science Park, Shatin, New Territories, Hong Kong (CN); CHEUNG, Chung Hong, Hong Kong Science Park, Shatin, New Territories Hong Kong (CN)
(74) Representative: Ipsilon
(86) International application number: PCT/CN2021/130403
(87) International publication number: WO 2022/151828

(57) **Abstract**

The invention provides a membranous immobilized cell, polypeptide, oligopeptide or protein and a preparation method thereof. The method comprises the following steps: 1) providing un-film-form chitosan, wherein the chitosan is un-pre-crosslinked or pre-crosslinked; 2) providing a mixture of the un-film-form chitosan and cell, polypeptide, oligopeptide or protein, and in the mixture, the un-film-form chitosan is in a dissolved state; 3) mixing the mixture with a crosslinking reagent to obtain a co-crosslinked product of the chitosan and the cell, polypeptide, oligopeptide or protein; 4) drying the co-crosslinked product to obtain membranous immobilized cell, polypeptide, oligopeptide or protein; wherein when un-pre-crosslinked chitosan is used in the step 1), the method further comprises the step 5) mixing the membranous immobilized cell, polypeptide, oligopeptide or protein with phosphate salt, so that chitosan molecules therein are crosslinked with each other. The method is a one-step method, the process is simple, time- and cost-saving.

## Description

### Field of the Invention

The invention belongs to the field of immobilized cells and proteins, and in particular relates to membranous immobilized cells, polypeptides, oligopeptides or proteins and a preparation method thereof.

### Background of the Invention

The application of enzymes is becoming more and more widespread. Due to the ease of separation from products and reusability, immobilized enzymes are often used in industrial applications to reduce production costs. There are many ways to immobilize enzymes, such as physical adsorption, affinity binding, covalent crosslinking, flocculation embedding, etc. (see literature "Roger A. Sheldon, 2007, Advanced Synthesis & Catalysis, 349: 1289-1307").

The production process of the existing carrier is complex, for example, the preparation of silicon carrier requires the use of various organic solutions (Zhongyi Yuan, Xingjia Wu, Shiyun Li, Xinsong Ji, "Preparation of a Chitosan Bead Carrier and Its Application to Enzyme Immobilization method", Chinese patent application publication number: CN1407103A); the preparation of porous composite oxides requires the use of high temperature and high pressure (Haozhen Quan, Shunjiao Hong, Guidong Bai, Huizhen Jin, Dongkun Pu, " Preparation of Porous Composite Oxide", Chinese Patent Application Publication No.: CN1171295A). Generally, gel carrier preparation process is simple, the gel particles are formed by adding the gel solution dropwise with a syringe into the solution that can make it gel. For example, dropping sodium alginate into calcium chloride solution; dropping carrageenan into potassium chloride solution. However, this technology needs to design specific equipment for industrial production (Zhaojing Zeng, Pingkai Ouyang, Guangliang Pan, "Immobilized Cell Carrier Forming Machine", Chinese Patent Application Publication No.: CN2181511Y), which greatly increases the production costs. Therefore, an immobilization method with simple preparation and low production costs has always been the goal of researchers in this field.

### Summary of the invention

In order to solve the problems in the above-mentioned prior art, the present invention provides membranous immobilized cells, polypeptides, oligopeptides or proteins and a preparation method thereof.

Specifically, the present invention provides:
(1) A method for preparing membranous immobilized cell, polypeptide, oligopeptide or protein, comprising the following steps:
   1) providing un-film-form chitosan, wherein the chitosan is un-pre-crosslinked or pre-crosslinked;
   2) providing a mixture of the un-film-form chitosan and cell, polypeptide, oligopeptide or protein, wherein in the mixture, the un-film-form chitosan is in a dissolved state;
   3) mixing the mixture with a crosslinking reagent to obtain a co-crosslinked product of the chitosan and the cell, polypeptide, oligopeptide or protein;
   4) drying the co-crosslinked product to obtain membranous immobilized cell, polypeptide, oligopeptide or protein;
      wherein, when step 1) uses un-pre-crosslinked chitosan, the method further includes:
   5) mixing the membranous immobilized cell, polypeptide, oligopeptide or protein obtained in step 4) with phosphate salt, so that the chitosan molecules therein are crosslinked with each other.
(2) The method according to (1), wherein the pre-crosslinked chitosan is prepared by mixing phosphate salt with un-pre-crosslinked chitosan.
(3) The method according to (1) or (2), wherein the weight ratio of the phosphate salt to the un-pre-crosslinked chitosan is (0.1-20): 1.
(4) The method according to (1) or (2), wherein the phosphate salt is one or more selected from the group of polyphosphate salt, polymetaphosphate salt and pyrophosphate salt.
(5) The method according to (1), wherein the crosslinking reagent in step 3) includes polyaldehyde compounds and genipin.
(6) The method according to (1), wherein the un-film-form chitosan includes chitosan powder and chitosan solution.
(7) The method according to (1), wherein the un-film-form chitosan is chitosan powder, and step 2) includes,
   I) providing the mixture of chitosan powder and cell, polypeptide, oligopeptide or protein;
   II) dissolving the chitosan powder with acetic acid.
(8) The method according to (1), wherein in step 2), the weight ratio of the un-film-form chitosan to the cells is 1:(1-20).
(9) The method according to (1), wherein in step 2), the weight ratio of the un-film-form chitosan to the polypeptides, oligopeptides or proteins is (0.5-10): 1.
(10) The method according to (1), wherein in step 3), the weight ratio of the crosslinking reagent to the un-film-form chitosan in the mixture is (0.005-10):1.
(11) The method according to (1), wherein the protein includes an enzyme.
(12) The membranous immobilized cell, polypeptide, oligopeptide or protein prepared by the method described in any one of (1)-(1 1).

Comparing with the prior art, the present invention has the following advantages:
The invention innovatively proposes a one-step method to prepare membranous immobilized cell, polypeptide, oligopeptide or protein. "One-step method" refers to the simultaneous completion of the preparation of the membranous chitosan carrier and the immobilization of cells, polypeptides, oligopeptides or proteins, which reduces the cost and time of preparation of carrier. The preparation is simple and does not require the use of specific instruments, easy operation, suitable for industrial production.

In the method of the present invention, the loading capacity of cell, polypeptide, oligopeptide, protein or enzyme can be flexibly controlled by the amount of carrier used.

The main component of the carrier used in the present invention is chitosan, which is a natural non-toxic high molecular weight polymer and does not cause pollution to the environment.

The invention uses phosphate salt to crosslink the chitosan, which can effectively reduce the water swelling of membranous immobilized cell, polypeptide, oligopeptide, protein or enzyme. And phosphate salt is a kind of harmless food additive, safe and reliable.

### Brief Description of the Drawings

FIG. 1 shows sample 1 before soaking in deionized water in Experimental Example 1.
FIG. 2 shows sample 1 after soaking in deionized water in Experimental Example 1.
FIG. 3 shows sample 2 before soaking in deionized water in Experimental Example 1.
FIG. 4 shows sample 2 after soaking in deionized water in Experimental Example 1.
FIG. 5 shows sample 3 before soaking in deionized water in Experimental Example 1.
FIG. 6 shows sample 3 after soaking in deionized water in Experimental Example 1.
FIG. 7 shows sample 4 before soaking in deionized water in Experimental Example 1.
FIG. 8 shows sample 4 after soaking in deionized water in Experimental Example 1.
FIG. 9 shows sample 5 before soaking in deionized water in Experimental Example 1.
FIG. 10 shows sample 5 after soaking in deionized water in Experimental Example 1.

### Detailed Description of the Invention

The present invention will be further illustrated by the description of specific embodiment and with reference to accompanying figures, but this is not the limitation of the present invention. Any changes and modifications of the protocol according to the basic idea of the present invention is within the scope of the present invention, as long as the basic idea of the present invention is followed.

The invention provides a method for preparing membranous immobilized cell, polypeptide, oligopeptide or protein, comprising the following steps:
1) providing un-film-form chitosan, wherein the chitosan is un-pre-crosslinked or pre-crosslinked;
2) providing a mixture of the un-film-form chitosan and cell, polypeptide, oligopeptide or protein, wherein in the mixture, the un-film-form chitosan is in a dissolved state;
3) mixing the mixture with a crosslinking reagent to obtain a co-crosslinked product of the chitosan and the cell, polypeptide, oligopeptide or protein;
4) drying the co-crosslinked product to obtain membranous immobilized cell, polypeptide, oligopeptide or protein;
   wherein, when step 1) uses un-pre-crosslinked chitosan, the method also includes:
5) mixing the membranous immobilized cell, polypeptide, oligopeptide or protein obtained in step 4) with phosphate salt, so that the chitosan molecules in the membranous immobilized cell, polypeptide, oligopeptide or protein are crosslinked with each other.

The present invention utilizes the film-forming property of chitosan to prepare membranous immobilized cell, polypeptide, oligopeptide or protein, wherein the carrier is membranous chitosan, that is, chitosan membrane. Herein, a membranous immobilized cell, polypeptide, oligopeptide or protein is an immobilized cell, polypeptide, oligopeptide or protein membrane, and the two terms are used interchangeably.

Usually, the method for preparing immobilized cell or enzyme is to prepare a carrier first, and then immobilize the cell or enzyme on the carrier chemically or physically. The present invention breaks the inherent thinking, makes the preparation of the membranous chitosan carrier and the immobilization of cell, polypeptide, oligopeptide or protein complete at the same time, and realizes the process (carrier preparation and immobilization) that usually needs to be completed in two steps in one step to reduce the cost and time of preparation of carrier.

On the other hand, most of the film-forming materials have the problem of water swelling after film formation. Crosslinking film-forming materials helps to reduce the water swelling, but commonly used crosslinking reagents (such as epichlorohydrin) have certain toxicity, so the industrial application is greatly limited. The present invention selects and uses the phosphate salt which not only solve the problem of water swelling after film formation, but also is an environmentally friendly material.

The structure of the term "chitosan" as used herein is known in the art, and it is an aminopolysaccharide that is water insoluble but soluble in acetic acid.

The term "un-film-form chitosan" as used herein refers to the state that the chitosan is not membranous, that is, the chitosan is not in a form of membranous carrier required for immobilizing cells, polypeptides, oligopeptides or proteins. The un-film-form chitosan may be, for example, chitosan powder or chitosan solution. Neither chitosan powder nor chitosan solution is a carrier for the immobilization of cells, polypeptides, oligopeptides or proteins.

The term "un-pre-crosslinked chitosan" as used herein refers to chitosan in its natural state which has not been treated with crosslinking reagents.

The term "pre-crosslinked chitosan" as used herein refers to chitosan which has been crosslinked intermolecularly between chitosan molecules by phosphate salts.

In the present invention, the degree of deacetylation of chitosan used is above 80%.

The term "co-crosslinked product" as used herein refers to product that crosslinking occurs between chitosan and cells, polypeptides, oligopeptides or proteins, so that cells, polypeptides, oligopeptides or proteins are immobilized on chitosan by covalent bond.

Since the present invention uses a one-step method for carrier preparation and immobilization, the loss of cell, protein or enzyme activity in the carrier preparation process needs to be considered. In order to reduce the impact of phosphate salt treatment on cells, proteins or enzymes, the present invention preferably pre-crosslinks chitosan with phosphate salts, and then uses the pre-crosslinked chitosan to prepare membranous immobilized cells, proteins or enzymes.

Pre-crosslinked chitosan can be provided by intermolecular crosslinking of chitosan by the reaction of un-pre-crosslinked chitosan with phosphate salts, wherein the un-pre-crosslinked chitosan is mixed with phosphate salts at room temperature for appropriate time, for example 30 minutes.

Preferably, the phosphate salt is one or more selected from the group of polyphosphate salt, polymetaphosphate salt or pyrophosphate salt.

The polyphosphate salt is preferably sodium tripolyphosphate.

The polymetaphosphate salt is preferably sodium trimetaphosphate or sodium hexametaphosphate.

The pyrophosphate salt is preferably sodium pyrophosphate.

Preferably, the crosslinking reagent described in step 3) is polyaldehyde compound or genipin.

The polyaldehyde compound includes glutaraldehyde and dialdehyde starch.

In a preferred embodiment, the un-film-form chitosan is chitosan powder, and step 2) includes,
I) providing the mixture of chitosan powder and cells, polypeptides, oligopeptides or proteins;
II) dissolving the chitosan powder with acetic acid.

Preferably, the final concentration of acetic acid is 0.5%-10% (v/v).

It is also possible to dissolve the chitosan powder with acetic acid first, and then mix the dissolved chitosan with the cell, polypeptide, oligopeptide or protein solution. However, the embodiment of mixing first and then dissolving can fully mix chitosan and cells, and reduce the overall volume, which is more conducive to industrial production.

In step 2), the weight ratio of the un-film-form chitosan to the cell is preferably 1:(1-20); the weight ratio of the un-film-form chitosan and the polypeptide, oligopeptide or protein is preferably (0.5-10):1.

In step 3), the weight ratio of the crosslinking reagent to the un-film-form chitosan in the mixture is preferably (0.005-10): 1. Step 3) can be performed at room temperature for appropriate time, for example 10 minutes.

In step 5), the weight ratio of the phosphate salt to the un-pre-crosslinked chitosan is preferably (0.1-20):1.

In the case of pre-crosslinking, the weight ratio of phosphate salt to un-pre-crosslinked chitosan is preferably (0.1-20): 1.

In a specific embodiment, the method of the present invention comprises the following steps performed in sequence:
a) mixing un-pre-crosslinked chitosan powder with cell, polypeptide, oligopeptide or protein solution;
b) adding acetic acid to dissolve the chitosan;
c) adding polyaldehyde compound to crosslink the chitosan with cells, polypeptides, oligopeptides or proteins;
d) drying the above mixture to obtain immobilized cell, polypeptide, oligopeptide or protein membrane;
e) mixing phosphate salt with the above-mentioned immobilized cell, polypeptide, oligopeptide or protein membrane to crosslink chitosan;
f) washing the immobilized cell, polypeptide, oligopeptide or protein membrane with deionized water, and drying to obtain the final immobilized cell, polypeptide, oligopeptide or protein membrane.

In another specific embodiment, the method of the present invention comprises the following steps performed in sequence:
A) Preparing pre-crosslinked chitosan:
   a) mixing un-pre-crosslinked chitosan powder with phosphate salt solution, making it react with phosphate salt to crosslink;
   b) filtering and washing the reacted chitosan powder;
   c) drying to obtain pre-crosslinked chitosan powder;
B) mixing pre-crosslinked chitosan powder with cell, polypeptide, oligopeptide or protein solution;
C) adding acetic acid to dissolve the chitosan;
D) adding polyaldehyde compound to crosslink the chitosan with cell, polypeptide, oligopeptide or protein;
E) drying the above mixed solution to obtain immobilized cell, polypeptide, oligopeptide or protein membrane.

In the present invention, the protein includes an enzyme.

The present invention also provides membranous immobilized cells, polypeptides, oligopeptides or proteins prepared by the method of the present invention, that is, immobilized cell, polypeptide, oligopeptide or protein membranes.

The present invention is further illustrated by the examples below, but these examples should not be construed as limiting the protection scope of the present invention.

### Example

Unless otherwise specified, the experimental methods used in the following examples are all performed using conventional experimental procedures, operations, materials and conditions in the art.

### Example 1: Preparation of membranous immobilized E. coli cells containing expressed glucose isomerase with un-pre-crosslinked chitosan followed by treatment of sodium trimetaphosphate.

1 g of *E.coli* cells containing expressed glucose isomerase (the preparation method of the *E. coli* was as described in the Chinese patent application with publication number CN1982445A) was resuspended in 9 ml of deionized water. 0.1 g of chitosan powder (Weifang Kehai Chitin Co., Ltd., degree of deacetylation of 95%) was added after the cells were completely resuspended. 50 µl of acetic acid was added under stirring, followed by addition of 0.25 ml of 1% (v/v) glutaraldehyde (Tianjin Damao Chemical Reagent Factory) solution. After reacting for 10 minutes, the mixed solution was placed in a petri dish to dry at room temperature. The dried mixture was soaked in 20 ml of 5% (w/v) sodium trimetaphosphate (pH 6.0) (Shanghai Aladdin Biochemical Technology Co., Ltd.) solution for 30 minutes. Unreacted sodium trimetaphosphate was removed with deionized water and dried at room temperature. 0.46 g of immobilized *E.coli* cells containing expressed glucose isomerase membrane was obtained.

10 mg of the membranous immobilized *E.coli* cells containing expressed glucose isomerase prepared by the above method was weighed. 1 ml of 45% glucose solution (containing 4 mM magnesium sulfate, 180 ppm sodium metabisulfite, pH 7.5) was added, and reacted in a shaking incubator for 10 minutes (60°C, the shaking speed was 1500 rpm). The reaction was terminated with ice water.

The resulting reaction mixture was diluted 10-fold with deionized water. The fructose concentration was detected by HPLC (WATERS HPLC collocated with Shodex SC1011 column and refractive index detector, with deionized water as mobile phase, flow rate was 1 ml/min, temperature column 80°C for sample separation).

It was found that, taking one micromole of fructose produced per minute as a unit, under the above reaction conditions, the activity of the prepared membranous immobilized *E. coli* cells containing expressed glucose isomerase was 182 U/g.

### Example 2: Preparation of immobilized glucose isomerase membrane with un-pre-crosslinked chitosan followed by treatment of sodium trimetaphosphate.

1 g of *E.coli* cells containing expressed glucose isomerase was resuspended in 4 ml of deionized water. After the cells were completely resuspended, the cells were broken using an ultrasonic cell disruptor in an ice bath (ultrasonic power 50 W, ultrasonic time 5 seconds, interval time 5 seconds, 30 cycles). The supernatant was obtained after centrifugation at 13,000 rpm for 20 minutes at 4°C.

The protein concentration of the supernatant was diluted to 10 mg/ml with deionized water. 0.1 g of chitosan powder was added to 10 ml of diluted supernatant. 50 µl of acetic acid was added while stirring, followed by addition of 0.25 ml of 1 % (v/v) glutaraldehyde solution. After reacting for 10 minutes, the mixed solution was placed in a petri dish to dry at room temperature. The dried mixture was soaked in 20 ml of 5% (w/v) sodium trimetaphosphate (pH 6.0) solution for 30 minutes. Unreacted sodium trimetaphosphate was removed with deionized water and dried at room temperature. 0.25 g of immobilized glucose isomerase membrane was obtained.

10 mg of the immobilized glucose isomerase membrane prepared by the above method was weighed and was tested in the same way as in Example 1, the activity of the immobilized glucose isomerase membrane was 193 U/g.

### Example 3: Preparation of immobilized glucose isomerase membrane with pre-crosslinked chitosan by sodium trimetaphosphate.

10 g of chitosan powder was taken. 400 ml of 1% sodium trimetaphosphate (pH 6.0) was added. The mixture was stirred at room temperature for 30 minutes. Unreacted sodium trimetaphosphate was removed with deionized water after filtration. 9.7 g of pre-crosslinked chitosan was obtained after drying at 60°C.

1 g of *E.coli* cells containing expressed glucose isomerase was resuspended in 4 ml of deionized water. After the cells were completely resuspended, the cells were broken using an ultrasonic cell disruptor in an ice bath (ultrasonic power 50 W, ultrasonic time 5 seconds, interval time 5 seconds, 30 cycles). The supernatant was obtained after centrifugation at 13,000 rpm for 20 minutes at 4°C.

The protein concentration of the supernatant was diluted to 10 mg/ml with deionized water. 0.1 g of pre-crosslinked chitosan powder was added to 10 ml of diluted supernatant. 50 µl of acetic acid was added while stirring, followed by addition of 0.25 ml of 1 % (v/v) glutaraldehyde solution. After reacting for 10 minutes, the mixed solution was placed in a petri dish to dry at room temperature. 0.24 g of an immobilized glucose isomerase membrane was obtained.

10 mg of the immobilized glucose isomerase membrane prepared by the above method was weighed and was tested by the same method as in Example 1, the activity of the immobilized glucose isomerase membrane was 270 U/g.

### Example 4: Preparation of immobilized glucose isomerase membrane with pre-crosslinked chitosan by sodium pyrophosphate.

2 g of chitosan powder was taken. 80 ml of 1% sodium pyrophosphate (pH adjusted to 6) was added. The mixture was stirred at room temperature for 30 minutes. Unreacted sodium pyrophosphate was removed with deionized water after filtration. 1.9 g of pre-crosslinked chitosan was obtained after drying at 60°C.

1 g of *E.coli* cells containing expressed glucose isomerase was resuspended in 4 ml of deionized water. After the cells were completely resuspended, the cells were broken using an ultrasonic cell disruptor in an ice bath (ultrasonic power 50 W, ultrasonic time 5 seconds, interval time 5 seconds, cycle 30 times). The supernatant was obtained after centrifugation at 13000 rpm for 20 minutes at 4°C.

The protein concentration of the supernatant was diluted to 10 mg/ml with deionized water. 0.1 g of pre-crosslinked chitosan powder was added to 10 ml of diluted supernatant. 50 µl of acetic acid was added while stirring, followed by addition of 0.25 ml of 1% (v/v) glutaraldehyde solution. After reacting for 10 minutes, the mixed solution was placed in a petri dish to dry at room temperature. 0.23 g of an immobilized glucose isomerase membrane was obtained.

10 mg of the immobilized glucose isomerase membrane prepared by the above method was weighed and was tested by the same method as in Example 1, the activity of the immobilized glucose isomerase membrane was 250 U/g.

### Example 5: Preparation of immobilized glucose isomerase membrane with pre-crosslinked chitosan by sodium tripolyphosphate.

2 g of chitosan powder was taken. 80 ml of 1% sodium tripolyphosphate (pH adjusted to 6) was added. The mixture was stirred at room temperature for 30 minutes. Unreacted sodium tripolyphosphate was removed with deionized water after filtration. 1.9 g of pre-crosslinked chitosan was obtained after drying at 60°C.

1 g of *E.coli* cells containing expressed glucose isomerase was resuspended in 4 ml of deionized water. After the cells were completely resuspended, the cells were broken using an ultrasonic cell disruptor in an ice bath (ultrasonic power 50 W, ultrasonic time 5 seconds, interval time 5 seconds, cycle 30 times). The supernatant was obtained after centrifugation at 13000 rpm for 20 minutes at 4°C.

The protein concentration of the supernatant was diluted to 10 mg/ml with deionized water. 0.1 g of pre-crosslinked chitosan powder was added to 10 ml of the diluted supernatant. 50 µl of acetic acid was added while stirring, followed by addition of 0.25 ml of 1% (v/v) glutaraldehyde solution. After reacting for 10 minutes, the mixed solution was placed in a petri dish to dry at room temperature. 0.22 g of an immobilized glucose isomerase membrane was obtained.

10 mg of the immobilized glucose isomerase membrane prepared by the above method was weighed and was tested by the same method as in Example 1, the activity of the immobilized glucose isomerase membrane was 200 U/g.

### Experimental Example 1: Testing of water swelling reduction of chitosan crosslinked by sodium trimetaphosphate, sodium pyrophosphate and sodium tripolyphosphate.

1 g of *E.coli* cells containing expressed glucose isomerase was resuspended in 4 ml of deionized water. After the cells were completely resuspended, the cells were broken using an ultrasonic cell disruptor in an ice bath (ultrasonic power 50 W, ultrasonic time 5 seconds, interval time 5 seconds, cycle 30 times). The supernatant was obtained after centrifugation at 13000 rpm for 20 minutes at 4°C.

The protein concentration of the supernatant was diluted to 10 mg/ml with deionized water. 0.1 g of chitosan powder was added to 10 ml of diluted supernatant. 50 µl of acetic acid was added while stirring, followed by addition of 0.25 ml of 1% (v/v) glutaraldehyde solution. After reacting for 10 minutes, the mixed solution was placed in a petri dish and dried at room temperature. 0.24 g of immobilized glucose isomerase membrane was obtained (control).

The control immobilized glucose isomerase membrane prepared above (sample 1) and the immobilized glucose isomerase membranes prepared in Examples 2-5 (samples 2-5) were cut into 2 cm × 2 cm and were soaked in 20 ml of deionized water at room temperature for 30 minutes.

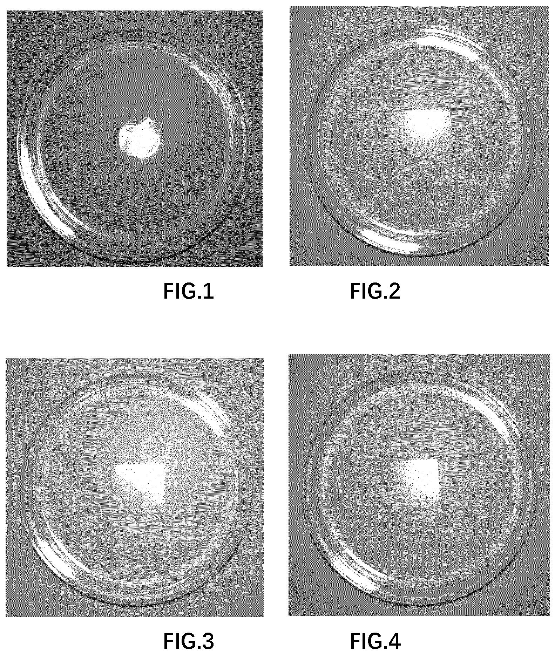

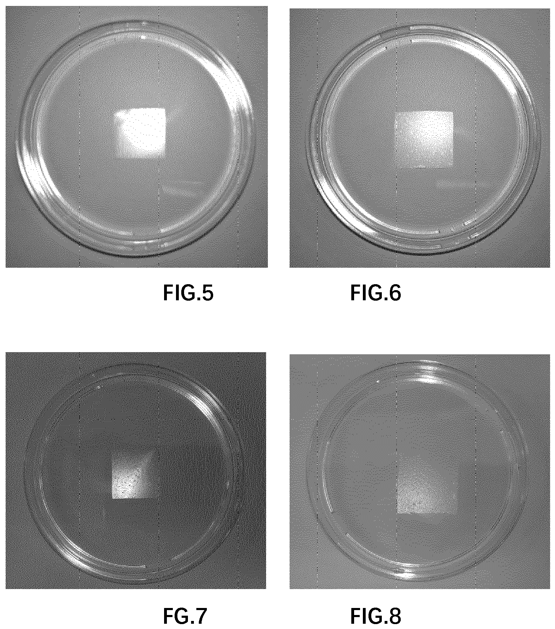

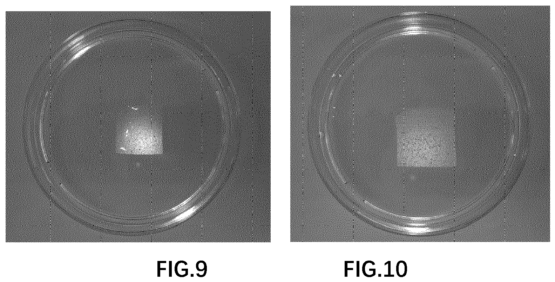

After soaking in deionized water, sample 1 was 2.7cm×2.7cm; sample 2 was 2cm×2cm; sample 3 was 2.3cm×2.3cm; sample 4 was 2. 5cm×2.5cm; sample 5 was 2.4cm×2.5cm (Fig. 1 to 10).

It was found that water swelling of the immobilized glucose isomerase membranes was effectively reduced by sodium trimetaphosphate, sodium pyrophosphate and sodium tripolyphosphate.

## Claims

1. A method for preparing membranous immobilized cell, polypeptide, oligopeptide or protein, comprising the following steps:
1) providing un-film-form chitosan, wherein the chitosan is un-pre-crosslinked or pre-crosslinked;
2) providing a mixture of the un-film-form chitosan and cell, polypeptide, oligopeptide or protein, wherein in the mixture, the un-film-form chitosan is in a dissolved state;
3) mixing the mixture with a crosslinking reagent to obtain a co-crosslinked product of the chitosan and the cell, polypeptide, oligopeptide or protein;
4) drying the co-crosslinked product to obtain membranous immobilized cell, polypeptide, oligopeptide or protein;
wherein, when step 1) uses un-pre-crosslinked chitosan, the method also includes:
5) mixing the membranous immobilized cell, polypeptide, oligopeptide or protein obtained in step 4) with phosphate salt, so that the chitosan molecules therein are crosslinked with each other.

2. The method according to claim 1, wherein the pre-crosslinked chitosan is prepared by mixing phosphate salt with un-pre-crosslinked chitosan.

3. The method according to claim 1 or 2, wherein the weight ratio of the phosphate salt to the un-pre-crosslinked chitosan is (0.1-20): 1.

4. The method according to claim 1 or 2, wherein the phosphate salt is one or more selected from the group of polyphosphate salt, polymetaphosphate salt and pyrophosphate salt.

5. The method according to claim 1, wherein the crosslinking reagent in step 3) includes polyaldehyde compounds and genipin.

6. The method according to claim 1, wherein the un-film-form chitosan includes chitosan powder and chitosan solution.

7. The method according to claim 1, wherein the un-film-form chitosan is chitosan powder, and step 2) comprises,
I) providing the mixture of chitosan powder and cell, polypeptide, oligopeptide or protein;
II) dissolving the chitosan powder with acetic acid.

8. The method according to claim 1, wherein in step 2), the weight ratio of the un-film-form chitosan to the cell is 1:(1-20).

9. The method according to claim 1, wherein in step 2), the weight ratio of the un-film-form chitosan to the polypeptide, oligopeptide or protein is (0.5-10):1.

10. The method according to claim 1, wherein in step 3), the weight ratio of the crosslinking reagent to the un-film-form chitosan in the mixture is (0.005-10):1.

11. The method according to claim 1, wherein the protein comprises an enzyme.

12. The membranous immobilized cell, polypeptide, oligopeptide or protein prepared by the method according to any one of claims 1-11.
